# EUROPEAN PATENT SPECIFICATION

(11) **EP 0 976 728 B1**
(45) Date of publication and mention of the grant of the patent: **04.12.2002**
(21) Application number: 99114247.2
(22) Date of filing: 28.07.1999
(51) Int. Cl.: C07C 323/25, C07C 319/14, C08G 18/77, G02B 1/04

(54) **Polyisocyanate compound, process for the production thereof and optical resin material comprising the same**
Polyisocyanatverbindungen, Verfahren zu ihrer Herstellung und diese enthaltende optische Harze
Composés de polyisocyanate, procédé pour leur préparation et résines optiques les contenant

(30) Priority: 29.07.1998 JP 21357098; 29.07.1998 JP 21357198
(43) Date of publication of application: 02.02.2000
(73) Proprietor: HOYA CORPORATION, Shinjuku-ku, Tokyo 161-8525 (JP)
(72) Inventor: Kitahara, Yoshitaka, c/o Hoya Corporation, Tokyo 161-8525 (JP); Jiang, Jian, c/o Hoya Corporation, Tokyo 161-8525 (JP)
(74) Representative: HOFFMANN - EITLE

(56) References cited:
- DATABASE CHEMABS [Online] CHEMICAL ABSTRACTS SERVICE, COLUMBUS, OHIO, US STN, CAPLUS, accession no. 1994:508012, XP002113928 & JP 06 065193 A (HOYA CORP)
- DATABASE CHEMABS [Online] CHEMICAL ABSTRACTS SERVICE, COLUMBUS, OHIO, US STN, CAPLUS, accession no. 1997:320993, XP002113929 & JP 09 071632 A (MITSUI TOATSU CHEMICALS INC)

## Description

### Technical Field of the Invention

The present invention relates to polyisocyanate compounds, a process for the production thereof and to an optical resin material comprising the novel polyisocyanate compounds.

More specifically, the present invention relates to a polyisocyanate compound useful as a starting material of an optical resin material which has excellent optical characteristics such as a high refractive index and a low dispersion, and a process for producing the same at good efficiency. Furthermore, it relates to an optical resin material comprising the polyisocyanate compound which has excellent optical characteristics such as a high refractive index, a low dispersion, an excellent transparency, lack of optical distortion and the like, and which is also good in a solvent resistance and a weatherability. This resin material is excellently suited as a material for an optical product formed therefrom, such as a lens, a prism, optical fibers, a substrate for a recording medium, a filter or the like, and also for other products which benefit from the optical properties of the resin, such as a glass, a vase or the like.

### Prior Art

In comparison with a glass, plastics are lightweight, hard to break, and easy to dye. Therefore, plastics have been applied, in recent years, to optical use in various lenses and the like. As optical plastic materials, polyethylene glycol bisallyl carbonate (CR-39) and polymethyl methacrylate (PMMA) have been generally used. However, these plastic materials have a low refractive index of 1.5 or less. Therefore, when they are used as lens materials, for example, the thickness of the lenses has to be increased with the increasing power. Consequently, not only the superiority of plastics regarding their low weight is impaired, but also the products obtained therefrom are not satisfactorily under aesthetic aspects. Furthermore, in concave lenses the particular problem arises that the circumferential thickness (edge thickness) is increased, which gives rise to increased birefringence and/or chromatic aberration.

In order to reduce the thickness of the lens, thus allowing to benefit as much as possible from the low specific gravity characteristics of plastics, a plastic material having a high refractive index has been in demand. As a material having such a performance, for example, (1) a polymer comprising a xylylene diisocyanate compound and a polythiol compound (official gazette of JP-A-63-46213), (2) a resin comprising an aliphatic linear sulfur-containing bifunctional isocyanate and a polythiol compound (official gazette of JP-A-2-153302), (3) a polymer comprising an aliphatic branched sulfur-containing bifunctional isocyanate compound and a polythiol (official gazette of JP-A-10-45707) are disclosed.

The above-mentioned polymer (1), wherein a limited range of compounds may be polymerized in combination with the polythiol compound has an increased refractive index. However, this polymer gives rise to the problems that the Abbe number is decreased and the chromatic aberration is increased.

Furthermore, the resins (2) and (3) have a high refractive index and are improved with regard to the chromatic aberration, but they still are insufficient in that the solvent resistance is poor.

Further, all these resins are uncrosslinked polymers obtained from bifunctional isocyanate compounds. Thus, a special crosslinking agent is needed separately to improve the solvent resistance. This leads to the problem that it is unavoidable to limit the type of the polythiol compound to be polymerized.

Japanese Patent Laid-Open No. 65193/1994 teaches the use of a compound of the general formula R1-R2(NCO)₂-R3-NCO, wherein R1 represents hydrogen, methyl, ethyl, R2 represents sulfur-substituted alkane-triyl or alkane tetrayl and R3 represents alkylene, for the preparation of a high-refractivity, low-dispersion polymer having good heat resistance, which can be used as an optical material.

JP 09 071632 discloses the preparation of transparent lenses using a polythiourethane, which is derived from tris (isocyanatomethylthio)methane as the isocyanate component.

### Object of the Invention

Based on the above circumstances, it is an object of the present invention to provide a polyisocyanate compound which can give an optical resin material having a high refractive index, a low dispersion and an excellent solvent resistance, and a process for producing this compound at good efficiency.

Intimately connected thereto it is another object of the present invention to provide an optical resin material comprising the polyisocyanate compound which has excellent optical characteristics such as a high refractive index, a low dispersion, an excellent transparency and lack of optical distortion, and which is also good in a solvent resistance and a weatherability.

### Means For Solving the Problem

The present inventors have assiduously conducted investigations to achieve the above-mentioned object, and have consequently found that polyisocyanate compounds having sulfur atoms contributing to a high refractive index and a low dispersion in the main skeleton and having three isocyanates as polymerization functional groups are capable to achieve the above object, and that these compounds can be obtained at good efficiency by a specific process.

In close relation thereto the present inventors further conducted investigations to develop the optical resin material having the above-mentioned desirable properties, and have consequently found that an optical resin material formed of a poly(thio)urethane obtainable by a polyaddition reaction of a component comprising the above polyisocyanate compound of the present invention with a component containing a compound having two or more groups, independently selected from hydroxyl and mercapto groups, in the molecule, can be adapted to the object.

These findings have led to the completion of the present invention.

That is, the present invention provides a polyisocyanate compound of the general formula (I) wherein n is an integer of 1 to 4, and each individual n represents the same value.

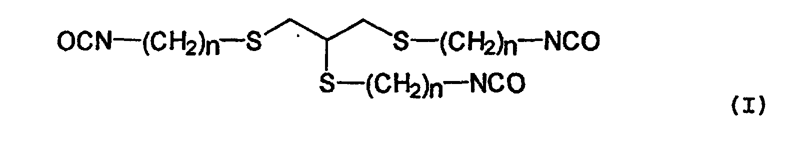

Further, this polyisocyanate compound can be produced by a process comprising the steps of
(1) preparing a tricarboxylic acid ester of the general formula (III) 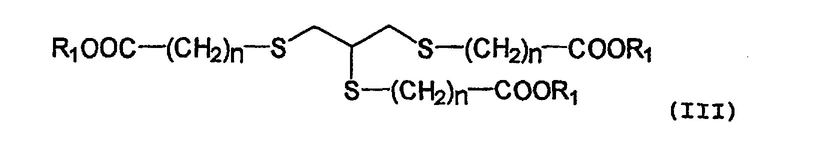 by reacting
   a) 1,2,3-trimercaptopropane with a halogeno-aliphatic carboxylic acid ester of the general formula (II)

      X-(CH₂)ₙ-COOR¹ (II)

      wherein R¹ is a lower alkyl group, X is a halogen atom and n is as defined above, or
   b) 1,2,3-trihalogenopropane with a mercapto aliphatic carboxylic acid ester of the general formula (V)

      HS-(CH₂)ₙ-COOR¹ (V)

      wherein R¹ is a lower alkyl group and n is as defined above, or
   c) 1,2,3-trimercaptopropane with an aliphatic unsaturated carboxylic acid lower alkyl ester of the general formula (VI)

      CH₂=CH-(CH₂)ₖ-COOR¹ (VI)
   wherein R¹ is a lower alkyl group and k is 0, 1 or 2,
(2) reacting compound (III) with hydrazine to obtain a tricarbonyl hydrazide of the general formula (IV) 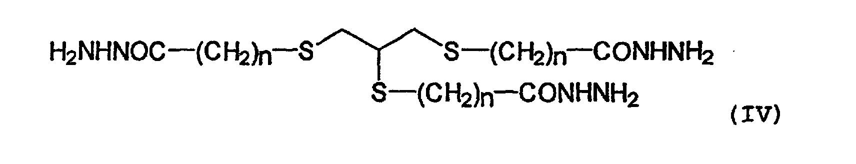
(3) converting the carbonyl hydrazide groups into isocyanate groups by reacting compound (IV) with nitrous acid.

According to the invention there is further provided an optical resin material formed of a poly(thio)urethane which is obtainable by polymerizing a monomer mixture comprising
(A) a component containing at least one polyisocyanate compound of the general formula (I) wherein n is an integer of 1 to 4, and each individual n represents the same value 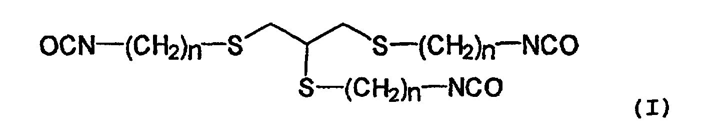
(B) a component containing at least one compound having two or more groups in the molecule each independently representing a hydroxyl group or a mercapto group.

### Brief Description of the Drawings

[Fig. 1] is an ¹H-NMR spectrum of 1,2,3-tris(isocyanatoethylthio)propane obtained in Production Example 1.
[Fig. 2] is an IR spectrum of 1,2,3-tris(isocyanatoethylthio)propane obtained in Production Example

### Mode For Carrying Out the Invention

### (1) The polyisocyanate compound

The polyisocyanate of the present invention is a novel compound represented by general formula (I) wherein n is an integer of 1 to 4, and each individual n represents the same value

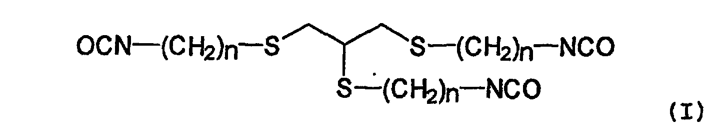

As is apparent from general formula (I), this novel compound has a basic skeleton structure wherein sulfur atoms are bound to the 1-, 2- and 3-positions of propane, respectively ,and furthermore three isocyanate groups are present in the molecule. Due to the presence of the sulfur atoms the refractive index and the Abbe number of the polyisocyanate compound itself are increased. Accordingly, when an optical resin material is produced using this polyisocyanate compound, the refractive index and the Abbe number of the optical resin material are also increased. Further, since this polyisocyanate compound has the three isocyanate groups, it becomes itself a crosslinking agent. Accordingly, when the optical resin material is produced using this polyisocyanate compound, not only a high solvent resistance but also a high heat resistance and excellent mechanical properties can be imparted to the optical resin material without adding an additional crosslinking agent as a secondary component. In this polyisocyanate compound, a compound in which n in general formula (I) is 1 or 2 is especially preferable.

As examples of the polyisocyanate compound represented by general formula (I), the following compounds may specifically be mentioned.

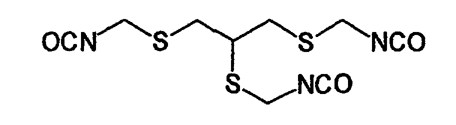

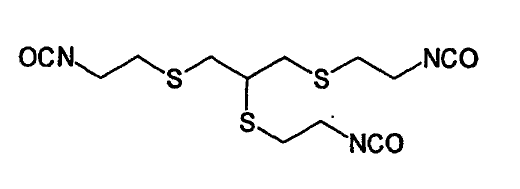

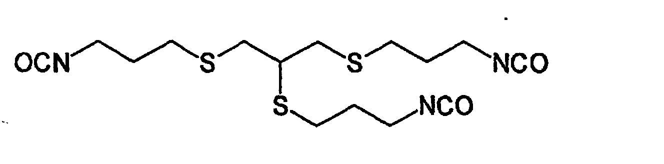

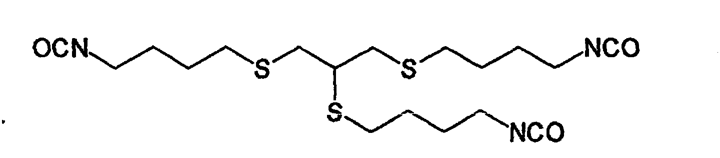

### (2) Process for producing the polyisocyanate compound

As a process for producing this polyisocyanate compound represented by general formula (I), a process leading to a polyisocyanate compound having the desired structure is in principle available in the state of art, and is not particularly limited. However, the polyisocyanate can be produced at especially good efficiency according to the following process of the present invention.

According to this process , in a first step a tricarboxylic acid ester of the general formula (III) is formed 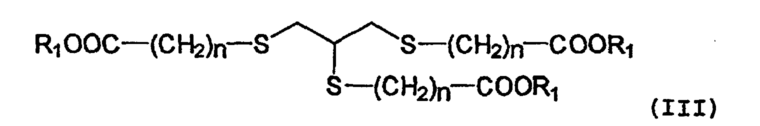 wherein R¹ represents a lower alkyl group, and n represents an integer of from 1 to 4, and each individual n represents the same value. This can be achieved by any one of three different reactions outlined below.
a) The first alternative is to react 1,2,3-trimercaptopropane with a halogeno-aliphatic carboxylic acid ester of the general formula (II)

   X-(CH₂)ₙ-COOR¹ (II)

   wherein R¹ is a lower alkyl group, X is a halogen atom and n is an integer of from 1 to 4.
   In this reaction, it is advisable that 1 mol of 1,2,3-trimercaptopropane is reacted with substantially 3 mol of the halogeno-aliphatic carboxylic acid lower alkyl ester represented by general formula (II) in the presence of a hydrogen halide trapping agent. In this case, an appropriate solvent can be used as required.
b) As a second option, 1,2,3-trihalogenopropane is reacted with a mercapto-aliphatic carboxylic acid ester of the general formula (V)

   HS-(CH₂)ₙ-COOR¹ (V)

   wherein R¹ is a lower alkyl group and n is an integer of from 1 to 4.
   In this reaction, it is advisable that 1 mol of the 1,2,3-trihalogenopropane is reacted with substantially 3 mol of the mercapto-aliphatic carboxylic acid lower alkyl ester represented by general formula (V) in the presence of a hydrogen halide trapping agent. In this case, an appropriate solvent can be used as required.
c) Finally, those compounds (III) wherein n is an integer of from 2 to 4 can be obtained by reacting 1,2,3-trimercaptopropane with an aliphatic unsaturated carboxylic acid lower alkyl ester of the general formula (VI)

   CH₂=CH-(CH₂)ₖ-COOR¹ (VI)

   wherein R¹ is a lower alkyl group and k is 0, 1 or 2,
   In this reaction, it is advisable that 1 mol of 1,2,3-trimercaptopropane is reacted with substantially 3 mol of the aliphatic unsaturated carboxylic acid lower alkyl ester represented by general formula (VI) in the presence of a radical or anionic catalyst. In this case, an appropriate solvent can be used as required.

In the second step of the process according to the present invention, the tricarboxylic acid ester of the formula (III) obtained in step 1 is reacted with hydrazine monohydrate or the like to convert the tricarboxylic acid ester into a tricarbonyl hydrazide of the general formula (IV) 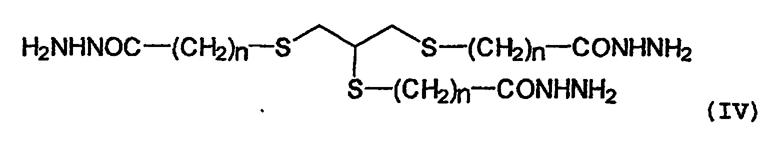 wherein n is as defined above).

At this time, a solvent such as a lower alcohol can be used as required.

Finally, the thus-obtained tricarbonyl hydrazide of the general formula (IV) is reacted with nitrous acid in, for example, a hydrochloric acid aqueous solution, and the reaction mixture is heated, thereby convert the carbonyl hydrazide groups into isocyanate groups.

Thus, the desired polyisocyanate compound represented by general formula (I) is obtained, wherein n is an integer of 1 to 4

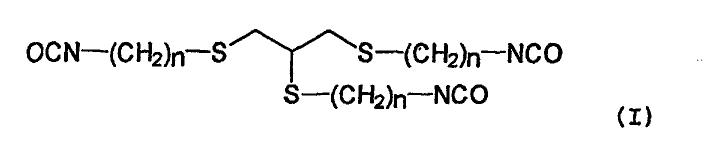

The lower alkyl group R¹ is not especially limited and any alkyl group may be used which allows for a reaction under reasonable reaction conditions. However, as examples of preferred alkyl groups a methyl group, an ethyl group, an n-propyl group, an isopropyl group may be mentioned.

Further, the polyisocyanate compound of the general formula (I) can also be produced by a phosgene method other than the above-mentioned processes of the present invention. This will be described by way of an example below.

First, 1 mol of the 1,2,3-trimercaptopropane is reacted with substantially 3 mol of a halogenoacetonitrile in the presence of a hydrogen halide trapping agent to obtain 1,2,3-tris(cyanomethylthio)propane of the formula (VII)

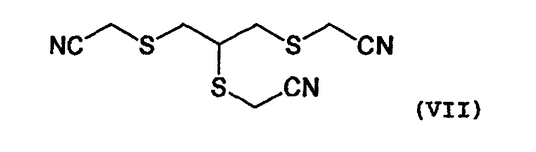

Subsequently, this is subjected to hydrogenation to lead it to 1,2,3-tris(aminoethylthio)propane of the general formula (VIII)

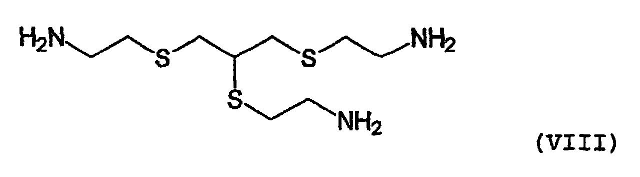

Then, the obtained compound it is reacted with phosgene, yielding the desired 1,2,3-tris(isocyanatoethylthio)propane of the formula (I-b)

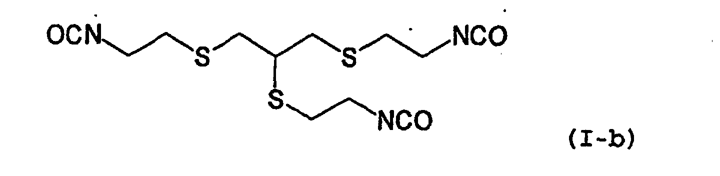

### (3) Optical resin material

The optical resin material of the present invention is formed of a poly(thio)urethane obtainable by polymerizing a composition comprising (A) a component containing at least one polyisocyanate compound of the above general formula (I), and (B) a component containing at least one compound selected from (a) a polythiol compound having two or more mercapto groups in the molecule, (b) a polyol compound having two or more hydroxy groups in the molecule, and (c) a compound having one or more mercapto groups and one or more hydroxy groups in the molecule.

As the component (A) as one of the starting materials of the poly(thio)urethane, a polyisocyanate compound represented by general formula (I) 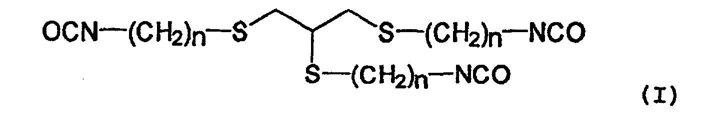 (wherein n represents an integer of from 1 to 4) is used.

As mentioned above, the specific structure of the compounds of the general formula (I), provides for the presence of each three sulfur atoms and three isocyanate groups in the molecule. Thus, the refractive index and the Abbe number of the polyisocyanate compound are increased. Furthermore, this polyisocyanate compound itself acts as a crosslinking agent. Therefore, the optical resin material produced therefrom also has an increased refractive index and Abbe number, and shows high solvent resistance, high heat resistance and excellent mechanical properties.

It is especially preferable that in the polyisocyanate compound of the general formula (I) n is 1 or 2.

As indicated, when the polyisocyanate compound represented by general formula (I) is used, there is no need to use a crosslinking agent, but a crosslinking agent can also be used conjointly as required.

As examples of the polyisocyanate compound represented by general formula (I), compounds having the following structures are specifically mentioned.

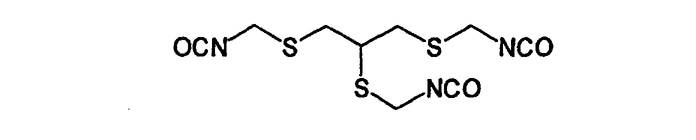

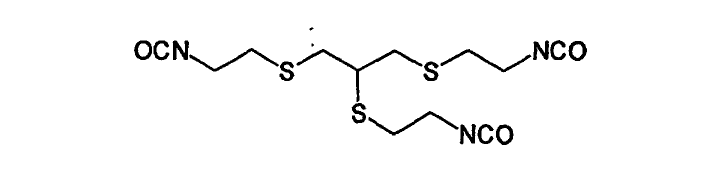

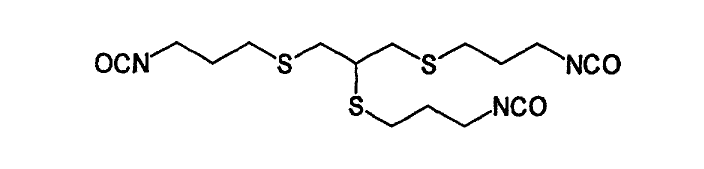

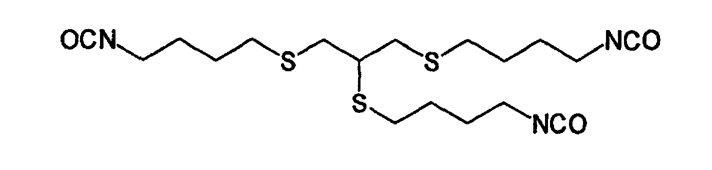

The component (A) may contain, to appropriately improve the properties of the optical resin material, one or two or more types of a compound having two or more isocyanate groups in a molecule, which differ from the polyisocyanate compound of the general formula (I).

Specific examples of these compounds include o-xylylene diisocyanate, m-xylylene diisocyanate, p-xylylene diisocyanate, α,α,α',α'-tetramethyl-p-xylylene diisocyanate, α,α,α',α'-tetramethyl-m-xylylene diisocyanate, 1,3,5-tris(isocyanatomethyl)benzene, hexamethylene diisocyanate, 1,4-diisocyanatobutane, isophorone diisocyanate, norbornene diisocyanate, bis(4,4'-isocyanatocyclohexyl)methane, 1,3-bis(isocyanatomethyl)cyclohexane, 1,3,5-tris (isocyanatomethyl)cyclohexane, 1,4-diisocyanatocyclohexane, 1,3,5-triisocyanatocyclohexane, lysine triisocyanate, 2,5-bis(isocyanatomethyl)-1,4-dithian, 1,3-dithiolan-4,5-diisocyanate, 4,5-bis(isocyanatomethyl)-1,3-dithiolan, isocyanatomethyl sulfide, 2-isocyanatoethyl sulfide, bis(isocyanatomethylthio)methane, 1,2-bis(isocyanatomethylthio)ethane, bis (2-isocyanatoethylthio)methane, 1,2-bis(2-isocyanatoethylthio)ethane, 1,7-diisocyanato-2,4,6-trithiaheptane, 1,5-diisocyanato-2-isocyanatomethyl-3-thiapentane and 1,4-diisocyanato-2-isocyanatomethyl-3-thiabutane.

The content of the polyisocyanate compound of the general formula (I) in the component (A) is preferably 0.1 mol-% or more, especially preferably 5 mol-% or more.

As the component (B), another starting material of the poly(thio)urethane, a component containing at least one compound having two or more groups in the molecule, each independently selected from hydroxyl groups and mercapto groups, is used.

Examples of compounds having two or more mercapto groups in the molecule include 2,5-bis (mercaptomethyl)-1,4-dithian, 2,5-bis(mercaptomethyl)-1,4-dithian dimer and polymer (trimer or higher polymer), 1,2,3-trimercaptopropane, tetrakis(7-mercapto-2,5-dithiaheptyl)methane, 1,2-ethanedithiol, 1,3-propanedithiol, tetrakismercaptomethylmethane, 2-mercaptoethyl sulfide; pentaerythritol tetrakismercaptopropionate, pentaerythritol tetrakismercaptoacetate, 1,2-benzenedithiol, 1,3-benzenedithiol, 1,4-benzenedithiol, 1,3,5-benzenetrithiol, 1,2-dimercaptomethylbenzene, 1,3-dimercaptomethylbenzene, 1,4-dimercaptomethylbenzene, 1,3,5-trimercaptomethylbenzene, toluene-3,4-dithiol, tris(3-mercaptopropyl) isocyanurate, 1,3-bis(mercaptomethyl)cyclohexane, 1,4-bis (mercaptomethyl) cyclohexane, 2,2-bis (mercaptomethyl)-1,3-propanedithiol, 1,2-bis(2-mercaptoethylthio)-3-mercaptopropane and 4,8-bis (mercaptomethyl) -3,6,9-trithia-1,11-undecanedithiol.

Further, examples of the compound comprised in (B) having two or more hydroxyl groups in the molecule include ethylene glycol, trimethylolpropane, glycerin, dihydroxybenzene, catechol, 4,4'-dihydroxyphenyl sulfide, 2-hydroxyethyl sulfide, bisphenol A·propylene oxide 5-mol adduct and glycerin·propylene oxide 3-mol adduct.

Besides, examples of the compound having one or more hydroxyl group(s) as well as one or more mercapto group(s) in the molecule include 2-mercaptoethanol, 2,3-dimercaptopropanol, 1,2-dihydroxy-3-mercaptopropane and 4-mercaptophenol.

As the compounds constituting the component (B), the compounds having two or more mercapto groups are preferable. Especially preferable are 2,5-bis(mercaptomethyl)-1,4-dithian represented by general formula (IX) (wherein p is an integer of from 1 to 20) and oligomers thereof

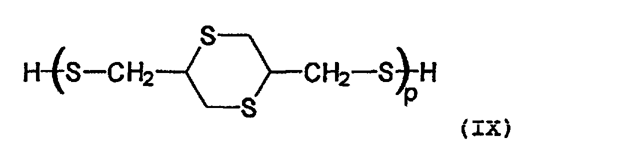

With respect to the ratio of the component (A) to the component (B) in the present invention, it is preferable that the molar ratio of the isocyanate group in the component (A) to the total amount of the mercapto group and the hydroxyl group in the component (B), [NCO]/([SH]+[OH]), is in the range of from 0.95 to 1.05.

The monomer mixture containing the component (A) and the component (B) may contain one, two or more types of the compound having two or more vinyl groups in the molecule to appropriately improve the properties and the like of the optical resin material in addition to the components (A) and (B). With respect to the ratio of these compounds used at this time, it is preferable that the (isocyanate group + vinyl group)/(mercapto group + hydroxyl group) molar ratio is in the range of from 0.95 to 1.5 and the (vinyl group)/(isocyanate group) molar ratio is 0.7 or less, and that the polymerizable functional groups contained in the component (B) are substantially all mercapto groups.

Specific examples of these compounds include 2,5-bis(2-thia-3-butenyl)-1,4-dithian, divinylbenzene, ethylene glycol di(meth)acrylate, polyethylene glycol di(meth)acrylate and a urethane-modified (meth)acrylate containing at least two (meth)acryloxy groups in the molecule. In the above-mentioned compounds (meth)acrylate means both of an acrylate and a methacrylate, and (meth)acryloxy group means both of an acryloxy group and a methacryloxy group.

To the optical resin material of the present invention can be added an ultraviolet absorber, a coloring matter, a pigment and the like for improving light absorption characteristics, an antioxidant, a coloration inhibitor and the like for improving a weatherability, and a release agent for improving a moldability, as required.

Examples of the ultraviolet absorber include benzotriazole type, benzophenone type, salicylic acid type UV-absorbers and the like. Examples of the coloring matter and the pigment include an anthraquinone type and an azo type.

Examples of the antioxidant and the coloration inhibitor include a monophenol type, a bisphenol type, a high-molecular phenol type, a sulfur type and a phosphorus type antioxidant. Examples of the release agent include a fluorine-type surfactant, a silicone-type surfactant, an acidic phosphate and a higher fatty acid.

Further, a catalyst may be used, as required, to improve a polymerization reactivity, and an amine compound and an organic metal compound are effective. Specific examples thereof include triethylenediamine, hexamethylenetetramine, N,N-dimethyloctylamine, N,N,N',N'-tetramethyl-1,6-diaminohexane, 4,4'-trimethylenebis (1-methylpiperidine), 1,8-diazabicyclo-(5,4,0)-7-undecene, dimethyltin dichloride, dimethyltin bis(isooctylthioglycolate), dibutyltin dichloride, dibutyltin dilaurate, dibutyltin maleate, dibutyltin maleate polymer, dibutyltin diricinoleate, dibutyltin bis(dodecylmercaptide), dibutyltin bis(isooctylthioglycolate), dioctyltin dichloride, dioctyltin maleate, dioctyltin maleate polymer, dioctyltin bis(butylmaleate), dioctyltin dilaurate, dioctyltin diricinoleate, dioctyltin dioleate, dioctyltin di(6-hydroxy)caproate, dioctyltin bis(isooctylthioglycokate), didodecyltin diricinoleate, copper oleate, copper acetylacetonate, iron acetylacetonate, iron naphthenate, iron lactate, iron citrate, iron gluconate, potassium octanate and 2-ethylhexyl titanate. The above-mentioned catalysts are effectively used either singly or in combination of two or more types.

In addition, when the vinyl compound is contained in the monomer mixture, the use of an organic peroxide, an azo compound or the like other than the above-mentioned catalyst is effective.

For example, the production of the optical resin material of the present invention using the polyisocyanate compound of the present invention is mentioned as follows. The uniform mixture of the component (A), the component (B), the additives and the catalyst is subjected to a known cast polymerization method, that is, it is cast into a die which is a combination of a glass or metal mold and a gasket made of a resin, and cured by heating. At this time, in order to expedite withdrawal of the resin after molding, the mold may previously be subjected to release treatment, or a release agent may be added to the mixture of the component (A) and the component (B). The polymerization temperature varies depending on the compound used. It is usually between -20 and +150°C. The polymerization time is generally between 0.5 and 72 hours. The optical resin material of the present invention can easily be dyed in water or an organic solvent using an ordinary disperse dye. At this time, for further expediting the dyeing, a carrier may be added or heating may be conducted.

The thus-obtained optical resin material is especially preferably used as a plastic lens, but is not limited thereto.

### Examples

Next, the present invention is illustrated more specifically by referring to Examples.

The properties of the resulting polyisocyanate compounds and optical resin materials (polymers) were evaluated according to the following methods.
(1) ¹H-NMR spectra (proton nuclear magnetic resonance spectra) were measured using an FT-NMR Device EX 270 Model supplied by JEOL.
(2) IR spectra (infrared absorption spectra) were measured using a MAGNA-IR Spectrometer 560 Model supplied by Nicoley.
(3) The refractive index (n_{D}) and Abbe number (ν_{D}) were measured at 20°C using a precision refractometer KPR-200 Model supplied by Kalnew.
(4) The appearance of the obtained products was visually observed.
(5) The weatherability was determined as follows:
   A lens (optical product using an optical resin material) was mounted on a weather meter fitted with a sunshine carbon arc lamp. When 200 hours passed, the lens was taken out, and the color thereof was compared with that of the lens before the test. The weatherability was evaluated according to the following standard.
   - (o):: unchanged
   - (∇):: slightly yellowed
   - (x):: yellowed
(6) To determine the solvent resistance a wiping test using acetone was conducted, and the solvent resistance was evaluated according to the following standard.
   - (o):: unchanged
   - (x):: The surface is roughened or swollen.
(7) The optical distortion was visually observed by the Schlieren method and was evaluated according to the following standard.
   - (o):: No distortion is observed.
   - (x):: Distortion is observed.

### Example 1 (Polyisocyanate Compound)

### Production of 1,2,3-tris(isocyanatoethylthio)propane

1,2,3-Trismercaptopropane (28.0 g, 0.20 mol) and 51.7 g (0.6 mol) of methyl acrylate were dissolved in 300 ml of chloroform. Triton B (40% by weight methanol solution, 0.6 g) was added as a catalyst in an ice bath, and the solution was stirred under reflux for 3 hours. The reaction solution was allowed to cool, then washed with a dilute sodium hydroxide aqueous solution and with water in this order, and dried over magnesium sulfate. Thereafter, chloroform was fully removed to obtain 71.5 g (0.18 mol) of colorless transparent 1,2,3-tris(methyloxycarbonylethylthio)propane.

This ester compound was dissolved in 50 ml of methanol, and added dropwise to a mixed solution of 81.0 g (1.62 mol) of hydrazine monohydrate and 90 ml of methanol at room temperature. After the completion of the dropwise addition, the mixture was stirred at 70°C for 4 hours. After the mixture was allowed to cool, white'crystals precipitated were collected through filtration, and recrystallized from methanol-water to obtain 69.4 g (0.17 mol) of 1,2,3-tris (hydrazinocarbonylethylthio) propane.

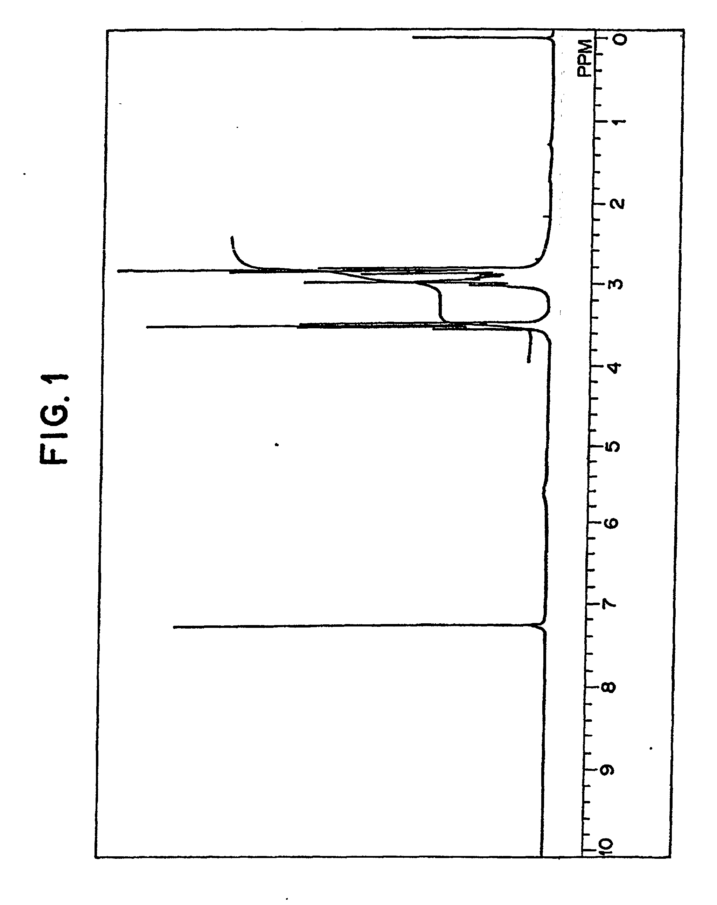

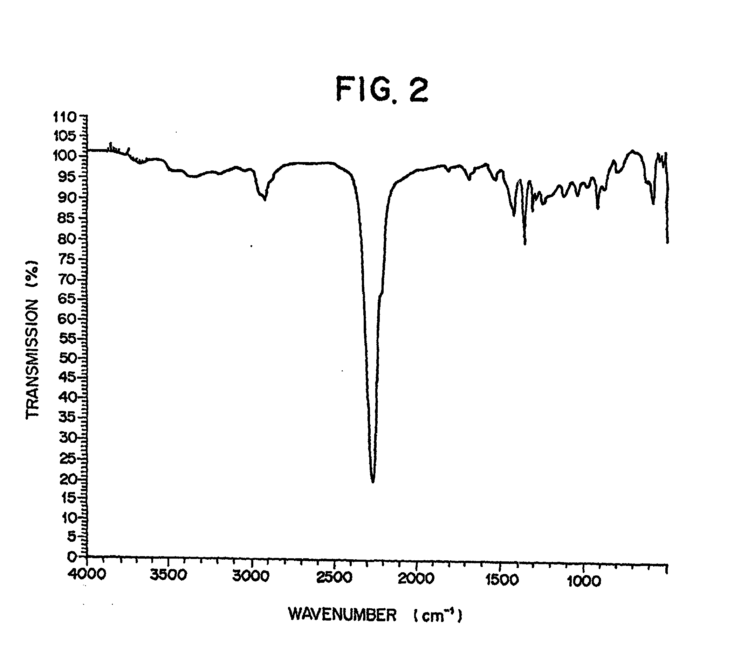

This hydrazide compound was dissolved in 280 g of a 7.2% by weight hydrochloric acid aqueous solution, and 36.1 g (0.52 mol) of sodium nitrite were added to a suspension with 160 ml of toluene. After the completion of the addition, the stirring was continued for 1 hour. The organic phase was extracted from the suspension, washed with water, dried (magnesium sulfate), and then heated to complete the transition reaction. Toluene as a solvent was fully removed from the reaction solution to obtain 46.0 g (0.13 mol) of a colorless transparent reaction product. This reaction product was identified to be a desired polyisocyanate compound from the ¹H-NMR spectrum and the IR spectrum. The ¹H-NMR spectrum of this novel polyisocyanate compound is shown in Fig. 1, and the IR spectrum thereof in Fig. 2.

### Example 2 (Optical Resin Material)

A mixture of 0.08 mol of 1,2,3-tris(isocyanatoethylthio)propane (designated SP-1 in Table 1) obtained in Example 1, 0.12 mol of 2,5-bis(mercaptomethyl)-1,4-dithian dimer (designated DBMD in Table 1) and 1.2 x 10⁻⁴ mol of dibutyltin dilaurate (designated DBTDL in Table 1) was uniformly stirred, and cast into glass molds for forming a lens. The mixture was polymerized at 50°C for 10 hours, then at 60°C for 5 hours and further at 120°C for 3 hours to obtain a plastic lens. The properties of the resulting plastic lens are shown in Table 1. From Table 1, it was found that the polymer obtained by using the polyisocyanate compound of Example 1 was colorless and transparent, the refractive index (n_{D}) was as high as 1.69, the Abbe number (ν_{D}) was also as high as 36 (low dispersion), the weatherability and the solvent resistance were excellent, and no optical distortion was observed.

### Examples 3 to 7

The same procedure as in Example 2 was conducted except using a monomer composition containing the polyisocyanate compound SP-1 [1,2,3-tris(isocyanatoethylthio)propane] obtained in Example 1 or 1,2,3-tris(isocyanatomethylthio)propane (designated SP-2 in Table 1) as shown in Table 1 to obtain a plastic lens. The properties of these plastic lenses were shown in Table 1. From Table 1, it was found that the resulting plastic lenses were colorless and transparent, the refractive index (n_{D}) was as high as between 1.65 and 1.70, the Abbe number (ν_{D}) was also as high as between 34 and 39 (low dispersion), the weatherability and the solvent resistance were excellent, and no optical distortion was observed.

### Comparative Example 1

A mixture of 0.06 mol of pentaerythritol tetrakismercaptopropionate (designated PETMP in Table 1), 0.12 mol of m-xylylene diisocyanate (designated XDI in Table 1) and 1.2 x 10⁻⁴ mol of dibutyltin dilaurate (designated DBTDL in Table 1) was uniformly stirred, and cast into glass molds for forming a lens. The mixture was heat-polymerized at 50°C for 10 hours, then at 60°C for 5 hours and further at 120°C for 3 hours to obtain a plastic lens. The properties of the resulting plastic lens are shown in Table 1. From Table 1, it was found that the plastic lens in Application Comparative Example 1 was colorless and transparent, no optical distortion was observed, and the solvent resistance was excellent, but the refractive index was as low as 1.59.

### Comparative Examples 2 and 3

The same procedure as in Comparative Example 1 was conducted except using monomer compositions shown in Table 1 to obtain plastic lenses. The properties of these plastic lenses were shown in Table 1. From Table 1, it was found that the plastic lens in Comparative Example 2 had a high refractive index of 1.68 and was excellent in the solvent resistance. However, it was colored yellow, the Abbe number was as low as 25, the weatherability was poor, and the optical distortion was observed. Further, the plastic lens in Comparative Example 3 was colorless and transparent, the refractive index was as high as 1.68, and no optical distortion was observed. However, the Abbe number was as low as 29, and the solvent resistance was also poor.

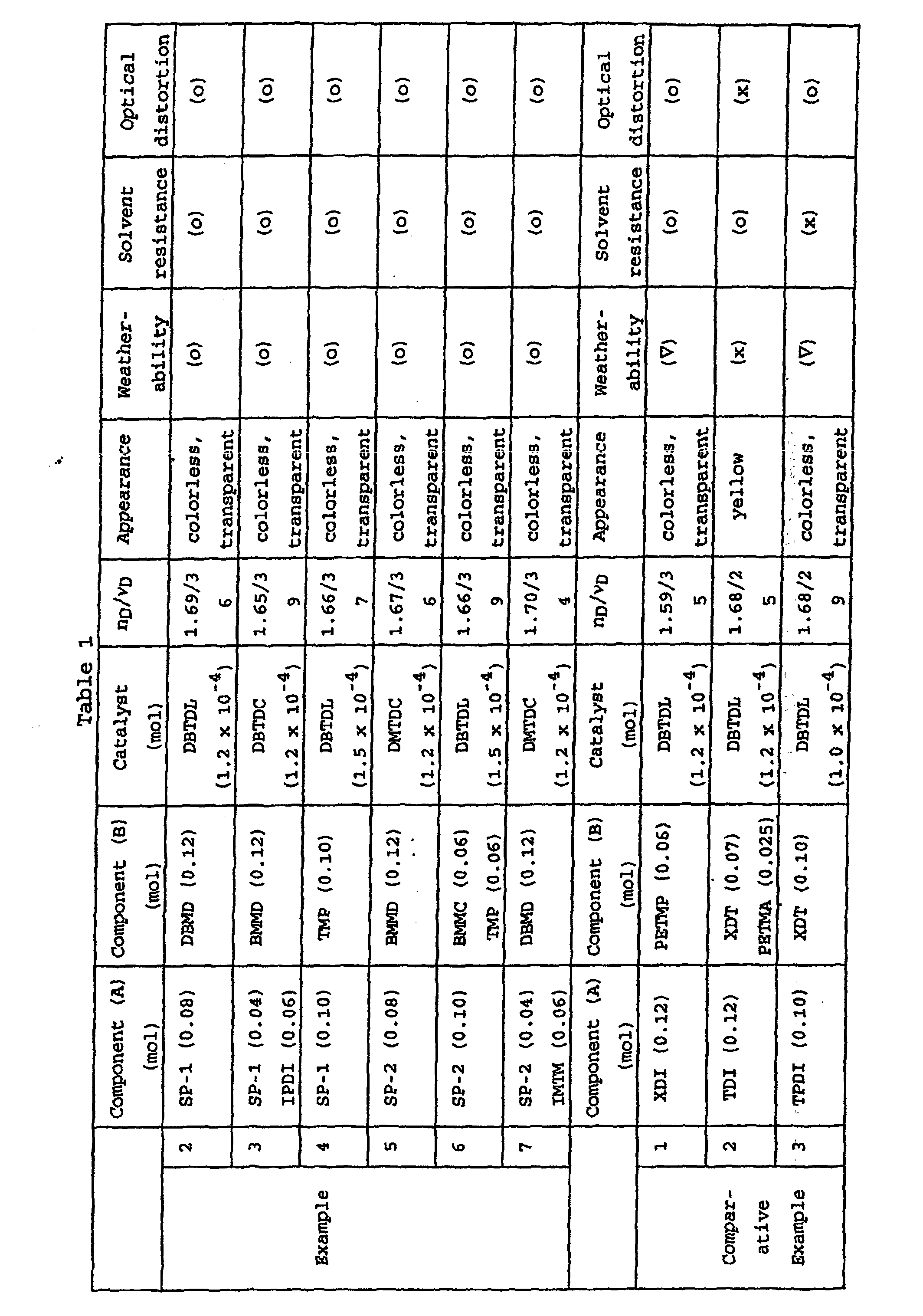

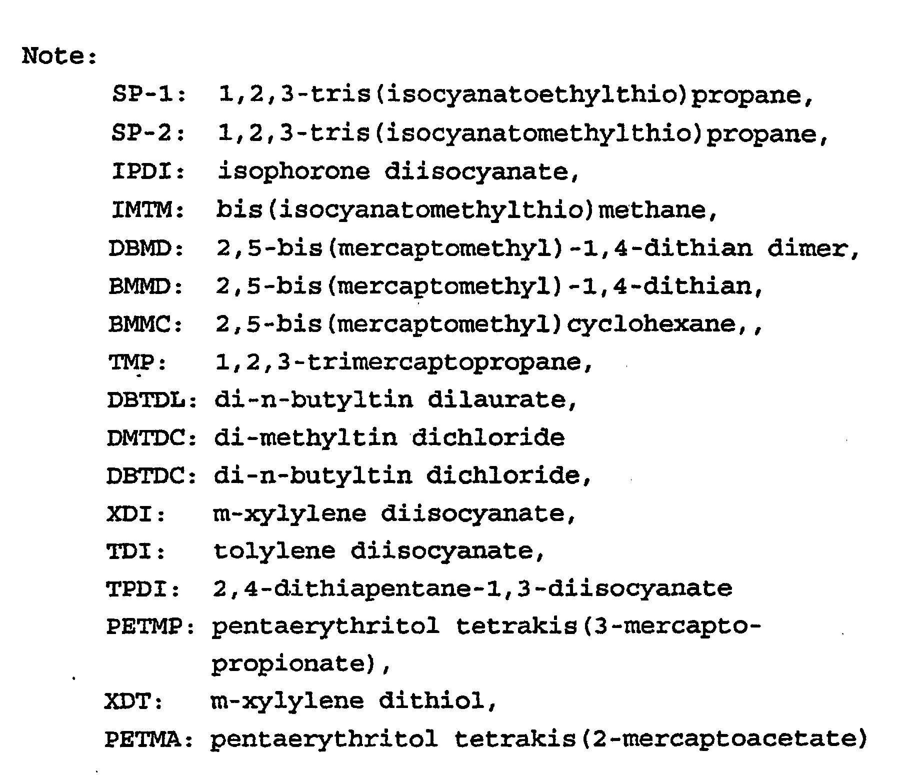

### Effects of the Invention

Since the polyisocyanate compound of the present invention has the aliphatic chain containing sulfur atoms as a basic skeleton, the refractive index and the Abbe number are high. It has three isocyanate groups, and is easily polymerized with at least one type of a compound having two or more hydroxyl groups in a molecule, a compound having two or more mercapto groups in a molecule and a compound having one or more hydroxyl groups and one or more mercapto groups in a molecule to provide a three-dimensionally crosslinked optical resin material.

In addition, since the optical resin material obtained by using this polyisocyanate compound contains the sulfur atoms in the main chain and is further crosslinked, the refractive index and the Abbe number are high, the weatherability, the solvent resistance and the transparency are excellent, and no optical distortion is observed.

Accordingly, it is preferably used in optical products, for example, lenses such as a spectacle lens, a camera lens and the like, a prism, optical fibers, substrates for recording medium used in an optical disk, a magnetic disk and the like, a filter and the like. Further, it is used in ornamental products such as a glass and a vase which are obtained by making the most of the property of the high refractive index.

## Claims

1. Polyisocyanate compound of the general formula (I) wherein n is an integer of 1 to 4, and each individual n represents the same value

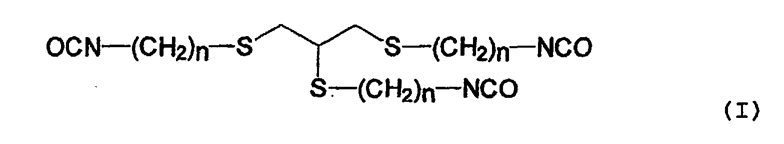

2. Polyisocyanate compound of claim 1 wherein n is 1 or 2

3. Process for the production of a compound of the general formula (I) 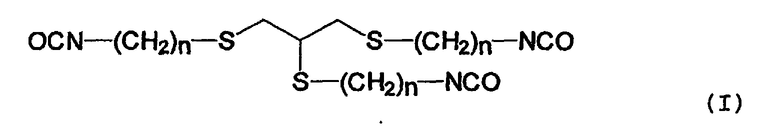 wherein n is an integer of 1 to 4, and each individual n represents the same value, comprising the steps of
(1) preparing a tricarboxylic acid ester of the general formula (III) 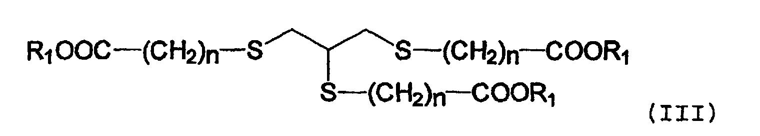 by reacting
a) 1,2,3-trimercaptopropane with a halogeno-aliphatic carboxylic acid ester of the general formula (II)
X-(CH₂)ₙ-COOR¹ (II)
wherein R¹ is a lower alkyl group, X is a halogen atom and n is as defined above, or
b) 1,2,3-trihalogenopropane with a mercapto-aliphatic carboxylic acid ester of the general formula (V)
HS-(CH₂)ₙ-COOR¹ (V)
wherein R¹ is a lower alkyl group and n is as defined above, or
c) 1,2,3-trimercaptopropane with an aliphatic unsaturated carboxylic acid lower alkyl ester of the general formula (VI)
CH₂=CH-(CH₂)ₖ-COOR¹ (VI)
wherein R¹ is a lower alkyl group and k is 0, 1 or 2,
(2) reacting compound (III) with hydrazine to obtain a tricarbonyl hydrazide of the general formula (IV) 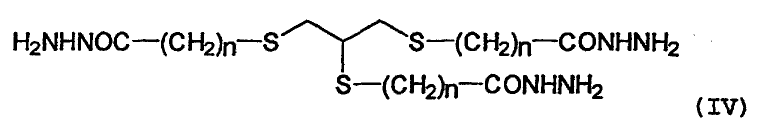
(3) converting the carbonyl hydrazide groups into isocyanate groups by reacting compound (IV) with nitrous acid.

4. Optical resin material formed of a poly(thio)urethane which is obtainable by polymerizing a monomer mixture comprising
(A) a component containing at least one polyisocyanate compound of the general formula (I) wherein n is an integer of 1 to 4, and each individual n represents the same value 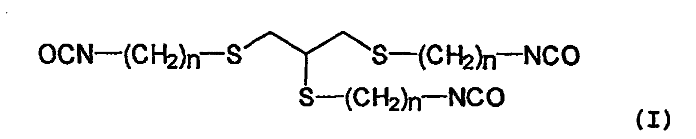
(B) a component containing at least one compound having two or more groups in the molecule each independently representing a hydroxyl group or a mercapto group.

5. Optical resin material according to claim 4, wherein in the polyisocyanate compound of the general formula (I) n is 1 or 2.

6. Optical resin material according to claim 4 or 5, wherein the polythiol (a) in the component (B) is a compound of the general formula (IX) wherein p is an integer of 1 to 20.

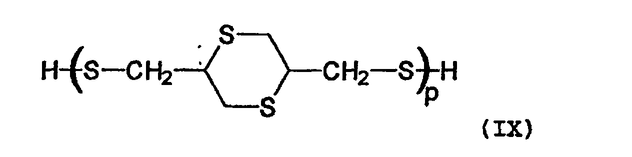

7. Optical resin material according to any of the claims 4 to 6, wherein the optical resin material has the form of a lens, a prism, an optical fiber, an optical filter or a substrate for a recording medium.

8. Use of a polyisocyanate compound according to claim 1 or 2 for the production of an optical resin material according to any of the claims 4 to 7.

9. Use of the optical resin material according to any of claims 4 to 7 for the production of an optical product.

10. Use according to claim 9 wherein the optical product is an optical element selected from a lens, a prism, an optical fiber, an optical filter or a substrate for a recording medium.

## Patentansprüche

1. Polyisocyanatverbindung der allgemeinen Formel (I), worin n eine ganze Zahl von 1 bis 4 ist und jedes individuelle n denselben Wert repräsentiert

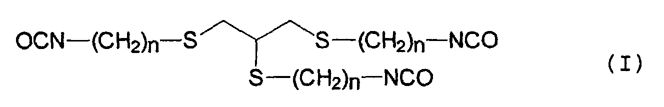

2. Polyisocyanatverbindung nach Anspruch 1, worin n 1 oder 2 ist.

3. Verfahren zur Herstellung einer Verbindung der allgemeinen Formel (I): 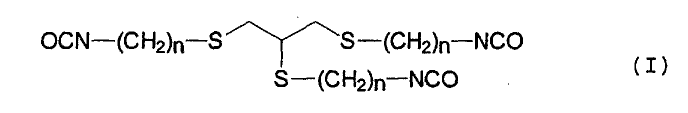 worin n eine ganze Zahl von 1 bis 4 ist und jedes individuelle n denselben Wert repräsentiert, umfassend die folgenden Schritte:
(1) Herstellen eines Tricarbonsäureesters der allgemeinen Formel (III): 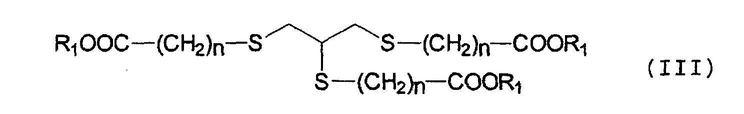 durch Umsetzen von
a) 1,2,3-Trimercaptopropan mit einem Halogenaliphatischen Carbonsäureester der allgemeinen Formel (II)
X-(CH₂)ₙ-COOR¹ (II)
worin R¹ eine Niederalkylgruppe ist, X ein Halogenatom ist und n wie oben definiert ist, oder
b) 1,2,3-Trihalogenpropan mit einem Mercaptoaliphatischen Carbonsäureester der allgemeinen Formel (V)
HS-(CH₂)ₙ-COOR¹ (V)
worin R¹ eine Niederalkylgruppe ist und n wie oben definiert ist, oder
c) 1,2,3-Trimercaptopropan mit einem aliphatischen ungesättigten Carbonsäureniederalkylester der allgemeinen Formel (VI)
CH₂=CH-(CH₂)ₖ-COOR¹ (VI)
worin R¹ eine Niederalkylgruppe ist und k 0, 1 oder 2 ist,
(2) Umsetzen der Verbindung (III) mit Hydrazin unter Erhalt eines Tricarbonylhydrazids der allgemeinen Formel (IV): 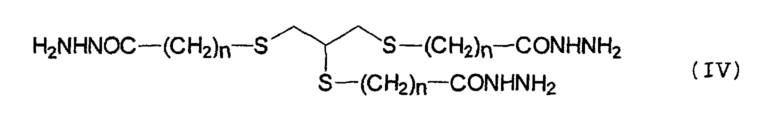
(3) Überführen der Carbonylhydrazidgruppen in Isocyanatgruppen durch Umsetzen der Verbindung (IV) mit salpetriger Säure.

4. Optisches Harzmaterial gebildet aus einem Poly(thio)urethan, welches erhältlich ist durch Polymerisierung einer Monomermischung umfassend:
(A) eine Komponente, die zumindest eine Polyisocyanatverbindung der allgemeinen Formel (I) enthält, worin n eine ganze Zahl von 1 bis 4 ist und jedes individuelle n denselben Wert repräsentiert 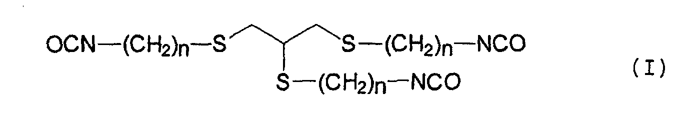
(B) eine Komponente enthaltend zumindest eine Verbindung mit zwei oder mehr Gruppen in dem Molekül, die jeweils unabhängig voneinander eine Hydroxylgruppe oder eine Mercaptogruppe repräsentieren.

5. Optisches Harzmaterial gemäß Anspruch 4, worin in der Polyisocyanatverbindung der allgemeinen Formel (I) n 1 oder 2 ist.

6. Optisches Harzmaterial gemäß Anspruch 4 oder 5, worin das Polythiol (a) in der Komponente (B) eine Verbindung der allgemeinen Formel (IX) ist, worin p eine ganze Zahl von 1 bis 20 ist:

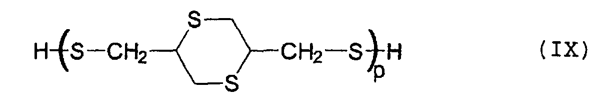

7. Optisches Harzmaterial gemäß einem der Ansprüche 4 bis 6, worin das optische Harzmaterial die Form einer Linse, eines Prismas, einer optischen Faser, eines optischen Filters oder eines Substrats für ein Aufzeichnungsmedium aufweist.

8. Verwendung der Polyisocyanatverbindung gemäß Anspruch 1 oder 2 zur Herstellung eines optischen Harzmaterials gemäß einem der Ansprüche 4 bis 7.

9. Verwendung des optischen Harzmaterials gemäß einem der Ansprüche 4 bis 7 zur Herstellung eines optischen Produkts.

10. Verwendung gemäß Anspruch 9, worin das optische Produkt ein optisches Element ausgewählt aus einer Linse, einem Prisma, einer optischen Faser, einem optischen Filter oder einem Substrat für ein Aufzeichnungsmedium ist.

## Revendications

1. Composé polyisocyanate selon la formule générale (I) où n est un entier de 1 à 4 et chaque n individuel représente la même valeur

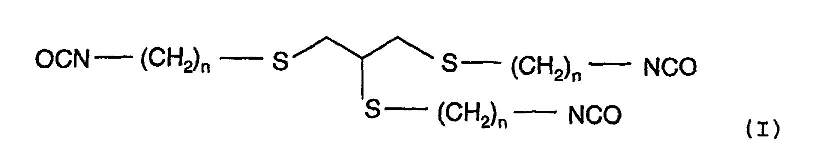

2. Composé polyisocyanate selon la revendication 1 où n est 1 ou 2.

3. Procédé de production d'un composé de formule générale (I) 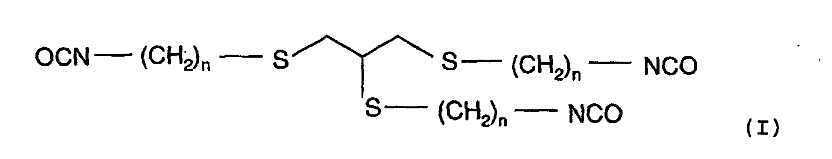 où n est un entier de 1 à 4, et chaque n individuel représente la même valeur incluant les étapes consistant à
(1) préparer un ester acide tricarboxylique de formule générale (III) 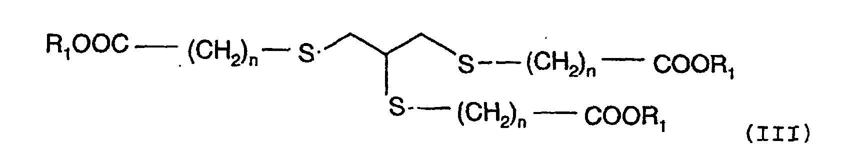 en faisant réagir
a) du 1,2,3-trimercaptopropane avec un ester d'acide carboxylique halogéno-aliphatique de formule générale (II)
X-(CH₂)ₙ-COOR¹ (II)
où R¹ est un groupe alkyle inférieur, X est un atome d'halogène et n est défini ci-dessus, ou
b) du 1,2,3-trihalogénopropane avec un ester d'acide carboxylique mercapto-aliphatique de formule générale (V)
HS-(CH₂)ₙ-COOR¹ (V)
où R¹ est un groupe alkyle inférieur et n est défini comme ci-dessus, ou
c) du 1,2,3-trimercaptopropane avec un ester inférieur alkyle d'acide carboxylique aliphatique non saturé de formule générale (VI)
CH₂=CH-(CH₂)ₖ-COOR¹ (VI)
où R1 est un groupe alkyle inférieur et k est égal à 0, 1 ou 2
(1) faire réagir le composé (III) avec de l'hydrazine pour obtenir un hydrazide tricarbonyle de formule générale (IV) 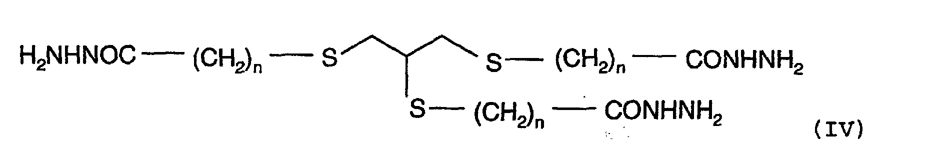
(2) transformer les groupes hydrazide carbonyle en groupes isocyanate en faisant réagir le composé (IV) avec un acide nitreux.

4. Matériel optique de résine formé d'un poly(thio)uréthane qui peut être obtenu en polymérisant un mélange monomère comprenant
(A) un composant contenant au moins un composé polyisocyanate de formule générale (I) où n est un entier de 1 à 4, et chaque n individuel représente la même valeur 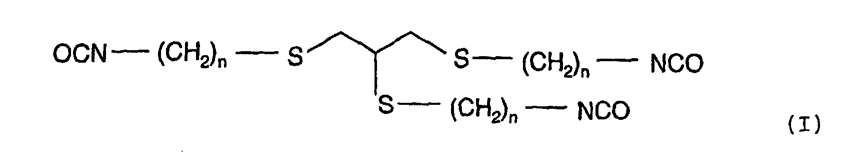
(B) un composant contenant au moins un composé ayant deux groupes ou plus dans la molécule chacun représentant indépendamment un groupe hydroxyle ou un groupe mercapto.

5. Matériel optique de résine selon la revendication 4, où dans le composé polyisocyanate de formule générale (I) n est 1 ou 2.

6. Matériel optique de résine selon la revendication 4 ou 5, où le polythiol (a) dans le composant (B) est un composé de formule générale (IX) où p est un entier de 1 à 20.

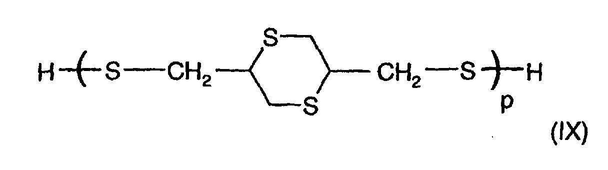

7. Matériel optique de résine selon l'une quelconque des revendications 4 ou 6, où le matériel optique de résine a la forme d'une lentille, d'un prisme, d'une fibre optique ou d'un substrat pour milieu d'enregistrement.

8. Utilisation d'un composé polyisocyanate selon la revendication 1 ou 2 pour la production de matériel optique de résine selon l'une quelconque des revendications 4 à 7.

9. Utilisation de matériel optique de résine selon l'une quelconque des revendications 4 à 7 pour la production d'un produit optique.

10. Utilisation selon la revendication 9 où le produit optique est un élément optique choisi parmi une lentille, un prisme, une fibre optique, un filtre optique ou un substrat pour milieu d'enregistrement.
